# EUROPÄISCHE PATENTSCHRIFT

(11) **EP 0 372 093 B1**
(45) Veröffentlichungstag und Bekanntmachung des Hinweises auf die Patenterteilung: **02.02.1994**
(21) Anmeldenummer: 89907882.8
(22) Anmeldetag: 06.06.1989
(51) Int. Cl.: A61M 25/00

(54) **EINRICHTUNG ZUR DIAGNOSTIK UND BEHANDLUNG VON NASENERKRANKUNGEN**
DEVICE FOR DIAGNOSING AND TREATING NASAL DISEASES
DISPOSITIF POUR DIAGNOSTIQUER ET TRAITER DES AFFECTIONS NASALES

(30) Priorität: 13.06.1988 SU 4432024; 13.06.1988 SU 4432025
(43) Veröffentlichungstag der Anmeldung: 13.06.1990
(73) Patentinhaber: YAROSLAVSKY MEZHOTRASLEVOI NAUCHNO-TEKHNICHESKY TSENTR, Yaroslavl, 150000 (SU)
(72) Erfinder: KOZLOV, Vladimir Sergeevich, Yaroslavl, 150000 (SU); MARKOV, Gennady Ivanovich, Yaroslavl, 150040 (SU); BUGROV, Vladimir Pavlovich, Yaroslavl, 150014 (SU)
(74) Vertreter: Füchsle, Klaus, Dipl.-Ing.
(86) Internationale Anmeldenummer: SU8900158
(87) Internationale Veröffentlichungsnummer: WO8912477

(56) Entgegenhaltungen:
- EP-A- 0 268 068
- WO-A-87/07510
- SU-A- 448 861
- SU-A- 627 828
- US-A- 4 198 981
- US-A- 4 655 746

## Beschreibung

### Gebiet der Technik

Die vorliegende Erfindung betrifft die Medizin und bezieht sich insbesondere auf eine Einrichtung zur Diagnostik und Behandlung von Nasenerkrankungen.

### Zugrundeliegender Stand der Technik

Es ist ein Endoskop zur Untersuchung von exkretorischen Öffnungen der Nasennebenhöhlen bekannt, mit dessen Hilfe man die sich zur Nasenhöhle hin öffnenden exkretorischen Öffnungen der Sinusse untersuchen und beim Ausfluss eines pathologischen Inhaltes aus ihnen eine Sinusitis diagnostizieren kann. Bei einer unvollständigen Füllung der Sinusse mit pathologischem Sekret oder bei einem dickflüssigen pathologischen Sekret findet jedoch keine Evakuierung desselben in die Nasenhöhle über die exkretorischen Öffnungen statt, was es nicht gestattet, mit Hilfe des Endoskops die Sinusitis zu diagnostizieren.

Bekannt ist auch eine Einrichtung, die ein mit einem elastischen Stab armiertes Rohr mit einem aufblasbaren Ballon und einer Manschette sowie mit drei Kanälen enthält, von denen zwei Kanäle getrennt mit dem Ballon und der Manschette verbunden sind, während der dritte Kanal eine Austrittsöffnung aufweist, die mit der Oberfläche des Rohres zwischen der Manschette und der Ballonflasche in Verbindung steht. Die Manschette und die Ballonflasche sind starr am biegsamen Rohr befestigt. Der dritte Kanal hat ein Übergangsstück für den Anschluss einer Spritze (s. z.B. SU, A, I3II7I4).

Bei Verwendung einer solchen Einrichtung führt man das armierte Rohr in die Nasenhöhle hinein, nimmt eine Isolierung, der Nasenhöhle seitens des Nasenloches und des Nasenrachenraums durch Aufblasen der Manschette und des Ballons vor. Man legt ein Vakuum an die so gebildete isolierte Nasenhöhle an, wodurch eine Entnahme des Inhaltes aus den Sinussen erfolgt. Bei der Verwendung dieser Einrichtung gelingt es jedoch nicht, zu bestimmen, aus welcher konkreten Nebenhöhle das Sekret evakuiert wird, und damit die Sinusitis topisch zu diagnostizieren.

Zudem ist aus der WO 87/07510 eine Einrichtung zur Diagnostik und Behandlung von Erkrankungen in Körperkanälen bekannt, mit einem länglichen, biegsamen Gehäuse, das drei zueinander konzentrische Kanäle aufweist. Der innere Kanal steht mit einem an einem der äußeren Enden des Gehäuses vorgesehenen, aufblasbaren Ballon in Verbindung, während über den äußeren Ringkanal eine zwischen den beiden Gehäuseenden befindliche, aufblasbare Manschette mit Luft befüllbar ist. Der mittlere Kanal ist im Raum zwischen Ballon und Manschette und zwar nahe dem Ballon, geöffnet und dient zur Einbringung in den Behandlungsraum von Luft und/oder Medikamenten. Durch die feste Beabstandung der Manschette vom Ballon und die feststehende Öffnung des mittleren Behandlungskanals am Gehäuseende kann diese Einrichtung nur zur Behandlung von Kanälen verwendet werden, die eine bestimmte Behandlungslage aufweisen und bei denen die Behandlungsmaterial-Einbringung nicht gezielt in eine bestimmte Richtung, vorzugsweise unter Sichtkontrolle und in unterschiedlichem Abstand von dem äußeren Ballon, angebracht werden kann.

### Offenbarung der Erfindung

Der Erfindung liegt die Aufgabe zugrunde, eine Einrichtung zur Diagnostik und Behandlung von unterschiedlichen Erkrankungen zu schaffen, die die Möglichkeit bietet, in der Nasenhöhle, Instrumente unterzubringen.

Die gestellte Aufgabe wird dadurch gelöst, dass in der Einrichtung zur Diagnostik und Behandlung von Nasen-Erkrankungen, die ein längliches biegsames Gehäuse, eine an diesem zwischen dessen Enden angeordnete aufblasbare Manschette enthält, wobei an einem dieser Enden ein aufblasbares Ballon angeordnet ist, der mit einem durchgehenden Längskanal in Verbindung steht, gemäss der Erfindung der Ballon an dem Gehäuse befestigt ist, die Manschette am Gehäuse längsverschiebbar angeordnet ist und ein Rohr zur Instrumentenaufnahme in der Manschette starr befestigt und abgedichtet ist, und die Einrichtung zur Diagnostik und Behandlung von Nasenerkrankungen bestimmt ist.

Solch eine Bau- und Anwendungsart art der erfindungsgemässen Einrichtung gibt die Möglichkeit, das Instrument, z.B. ein HNO-Fibroskop, das im Rohr untergebracht wird, an die Öffnung einer zu untersuchenden Höhle heranzubringen und diese unter gleichzeitiger Bildung einer isolierten Höhle, an die ein Vakuum anlegbar ist, sorgfältig zu untersuchen. Dadurch ist die Möglichkeit einer punktionslosen topischen Diagnostik von Sinusitiden gewährleistet.

Je nach der Situation kann im Rohr eine Spritze zur Absaugung eines pathologischen Inhaltes der Sinusse oder ein HNO-Fibroskop eingesetzt werden.

Bei der Diagnostik von Sinusitiden führt man über das in der Manschette befestigte Rohr das HNO-Fibroskop in die isolierte Höhle ein und untersucht die Öffnungen derSinusse, wodurch eine genaue topische Diagnostik erzielt wird.

### Kurzbeschreibung der Zeichnungen

Im weiteren wird die Erfindung an Hand von konkreten Ausführungsformen unter Bezugnahme auf die Zeichnung näher erläutert. Es zeigt:
Fig. I einen Längsschnitt der erfindungsgemässen Einrichtung zur Behandlung von Sinusitiden;

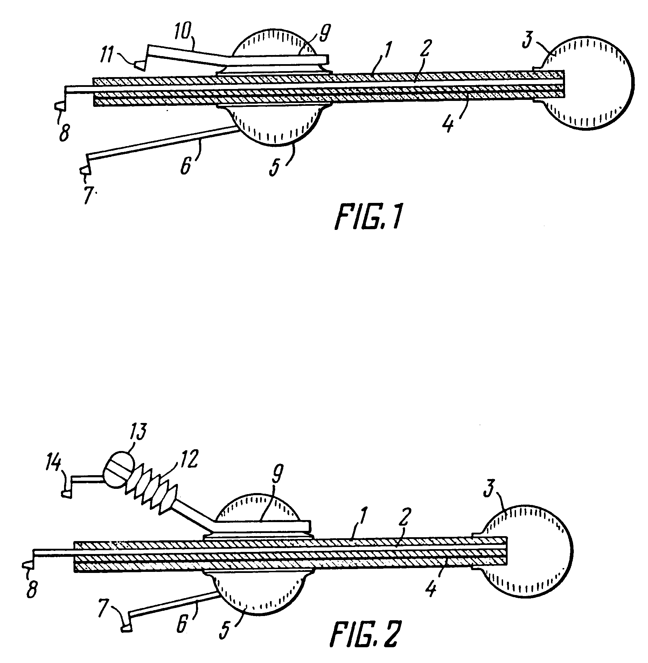

Fig. 2 eine andere Ausführungsform der erfindungsgemässen Einrichtung, welche im wesentlichen zur Diagnostik von Sinusitiden dient.

### Bevorzugte Ausführungsformen der Erfindung

Zuerst soll auf Fig. 1 eingegangen werden, in der zu erkennen ist, dass die Einrichtung zur Behandlung von Nasenerkrankungen und Nasennebenhöhlen ein längliches biegsames Gehäuse 1 enthält, das aus Latex ausgeführt ist. In diesem Gehäuse 1 ist ein durchgehender Kanal 2 vorhanden, der zur Luftzufuhr in den aufblasbaren Ballon 3 vorgesehen ist, der an einem Ende des Gehäuses angebracht ist. Das Gehäuse ist mit einem Stab 4 armiert, während an der Aussenseite zwischen den Enden des Gehäuses 1 eine aufblasbare Manschette 5 angeordnet ist, die mit einem Kanal 6 in Verbindung steht, der ein Ventil 7 aufweist. Es gibt ebenfalls ein Ventil 8 für den Kanal 2. In der Manschette 5 ist ein Rohr 9 starr befestigt, wobei die Einbettungsstellen des letzteren in der Manschette 5 hermetisiert sind, um ein Luftausströmen aus der Manschette bei deren Aufblasen zu vermeiden. Das Rohr 9 ist mit einem Übergangsstück 10 für eine Spritze (nicht gezeigt) und mit einem Stopfen 11 versehen.

Die beschriebene Einrichtung kann zur Behandlung einer Sinusitis wie folgt benutzt werden. Nach der in geeigneter Weise durchgeführten Anästhesie und Anämisierung reinigt man die Nasenhöhle durch Ausschneuzen. Entlang des unteren Teils des gemeinsamen Nasenganges führt man das Gehäuse in den Nasenrachenraum mit demjenigen Ende, an dem der aufblasbare Ballon 3 angebracht ist, ein, bis dieses Ballon die Choane erreicht. Alsdann führt man die Manschette 5 durch deren Verschiebung entlang des Gehäuses 1 in den Vorhof der Nase entsprechend den individuellen Abmessungen des gemeinsamen Nasenganges so ein, dass die Manschette vor dem Ende der unteren Nasenmuschel angeordnet ist. In das Ventil 8 steckt man eine Spritze ein, mit deren Hilfe über den Kanal 2 des Ballon 3 aufgeblasen wird, wodurch der Nasenrachenraum verschlossen wird. Dadurch wird ein geschlossener Raum gebildet, der die Nasenhöhle und die Sinusse umfasst. Darauf evakuiert man mit Hilfe einer Spritze über den Kanal des Rohres 9 und des Übergangsstück I0 deren Inhalt, nimmt eine Spülung vor und appliziert in bekannter Weise Arzneimittel.

Die in Fig. 2 gezeigte Einrichtung ist im wesentlichen analog der in Fig. 1 dargestellten nur mit dem Unterschied, dass die Einrichtung nach Fig. 2 zusätzlich mit einem Verlängerungsstück in Form eines Faltenbalges 12 verseheh ist, der mit einem Hermetisierelement 13, z.B. einem Ballon mit Druckluft, die über ein Ventil 14 zugeführt wird, abgeschlossen ist. Diese Einrichtung ist für die Diagnostik von Sinusitiden bestimmt. Die Wirkungsweise dieser Einrichtung ist analog der obenbeschriebenen, mit dem Unterschied, dass statt der Spritze, nach der Bildung eines geschlossenen Raums, durch das Hermetisierelement 13, den Faltenbalg 12 und den Kanal des Rohres 9 ein HNO-Fibroskop hineingeführt und an einen zu untersuchenden Sinus herangebracht wird. Über die im HNO-Fibroskop vorhandene Öffnung nimmt man eine Luftabsaugung vor, wodurch in der Nasenhöhle ein negativer Druck erzeugt wird. Dabei beginnt der pathologische Inhalt über die natürliche exkretorische Öffnung evakuiert zu werden, und wird durch das optische System des HNO-Fibroskops sichtbar gemacht. Dann wird das Fibroskop durch Ausdehnung des Faltenbalges an die Öffnungen der übrigen Sinusse der zu untersuchenden Nasenhälfte herangeführt. Aus den Sinussen, in denen ein pathologischer Inhalt festgehalten wurde, nimmt man eine Entnahme des Inhaltes über die Aspirationsöffnung des Fibroskops und dessen Analyse vor.

Also wird eine punktionslose hochpräzise topische Diagnostik und Behandlung gewährleistet, was in erheblichem Masse die Untersuchungs- und Behandlungsdauer verkürzt.

### Gewerbliche Anwendbarkeit

Die erfindungsgemässe Einrichtung zur Diagnostik und Behandlung von Sinusitiden kann aus Latex oder einem anderen biegsamen Material hergestellt sein. Sie kann sowohl zur Behandlung von Sinusitiden als auch zu anderen Zwecken, z.B. zum Stoppen einer Blutung verwendet werden.

## Patentansprüche

1. Einrichtung zur Diagnostik und Behandlung von Erkrankungen in Körperkanälen, umfassend ein längliches, biegsames Gehäuse (1), eine an diesem zwischen dessen Enden angeordnete aufblasbare Manschette (5), wobei an einem dieser Enden ein aufblasbarer Ballon (3) angeordnet ist, der mit einem durchgehenden Längskanal (2) in Verbindung steht,
**dadurch gekennzeichnet**, daß
a. der Ballon (3) an dem Gehäuse (1) befestigt ist;
b. die Manschette (5) an dem Gehäuse (1) längsverschiebbar angeordnet ist, und daß ein Rohr (9) zur Instrumentenaufnahme in der Manschette (5) starr befestigt und abgedichtet ist; wobei
c. die Einrichtung zur Diagnostik und Behandlung von Nasenerkrankungen bestimmt ist.

2. Einrichtung nach Anspruch 1, dadurch gekennzeichnet, daß das in der Manschette (5) befestigte Rohr (9) auf der dem aufblasbaren Ballon (3) abgewandten Seite mit einem hohlen Verlängerungsstück (12) versehen ist.

3. Einrichtung nach Anspruch 2, dadurch gekennzeichnet, daß das Verlängerungsstück (12) als Faltenbalg ausgebildet ist.

## Claims

1. Device for the diagnosis and treatment of disorders of bodily canals, comprising an elongate, flexible housing (1), an inflatable collar (5) arranged thereon between the ends thereof, with an inflatable balloon (3) being arranged on one of the said ends and connected to a longitudinal through-channel (2),
characterised in that
a. the balloon (3) is fixed on the housing (1);
b. the collar (5) is arranged in longitudinally displaceable manner on the housing (1), and that a tube (9) for receiving instruments is fixed in rigid manner in the collar (5) and is sealed;
c. the device is intended for the diagnosis and treatment of nasal disorders.

2. Device according to Claim 1, characterised in that the tube (9) fixed in the collar (5) is provided on the side remote from the inflatable balloon (3) with a hollow extension piece (12).

3. Device according to Claim 2, characterised in that the extension piece (12) is constructed as a bellows.

## Revendications

1. Dispositif pour le diagnostic et le traitement d'affections dans des canaux corporels, comprenant un boîtier long et flexible (1), un manchon gonflable (5) agencé entre les extrémités de celui-ci, un ballon gonflable (5) étant agencé à l'une de ces extrémités, qui est en liaison avec un canal continu (2),
caractérisé en ce que
a. le ballon (3) est fixé au boîtier (1) ;
b. le manchon (5) est agencé en étant longitudinalement mobile sur le boîtier (1) et en ce qu'un tube (9) pour recevoir des instruments est fixé rigidement dans le manchon (5) et est étanchéifié ;
c. le dispositif étant déterminé pour le diagnostic et le traitement d'affections nasales.

2. Dispositif selon la revendication 1, caractérisé en ce que le tube (9) fixé dans le manchon 5 est pourvu, sur son côté tourné vers le ballon gonflable (3), d'un prolongateur creux (12).

3. dispositif selon la revendication 2, caractérisé en ce que le prolongateur (12) a la forme d'un sac à pliage en accordéon.
